Europäisches Patentamt

European Patent Office

Office européen des brevets

(19)

(11) EP 0 844 980 B1

(12) EUROPÄISCHE PATENTSCHRIFT

(45) Veröffentlichungstag und Bekanntmachung des Hinweises auf die Patenterteilung:
06.12.2000 Patentblatt 2000/49

(21) Anmeldenummer: 96928427.2

(22) Anmeldetag: 08.08.1996

(51) Int Cl.7: C01G 23/053, C09C 1/36, A61K 7/00

(86) Internationale Anmeldenummer:
PCT/EP96/03504

(87) Internationale Veröffentlichungsnummer:
WO 97/07058 (27.02.1997 Gazette 1997/10)

(54) **HYDROLYTISCHE HERSTELLUNG VON TITANDIOXID-PIGMENTEN**

HYDROLYTIC PREPARATION OF TITANIUM DIOXIDE PIGMENTS

PREPARATION PAR VOIE HYDROLYTIQUE DE PIGMENTS A BASE D'OXYDE DE TITANE

(84) Benannte Vertragsstaaten:
DE ES FR GB IT

(30) Priorität: 19.08.1995  DE 19530574

(43) Veröffentlichungstag der Anmeldung:
03.06.1998  Patentblatt 1998/23

(73) Patentinhaber: BASF AKTIENGESELLSCHAFT
67056 Ludwigshafen (DE)

(72) Erfinder:
• JACHOW, Harald
  D-64625 Bensheim (DE)
• SCHWAB, Ekkehard
  D-67434 Neustadt (DE)
• KORMANN, Claudius
  D-67105 Schifferstadt (DE)
• DAUSCH, Wilma
  D-67117 Limburgerhof (DE)
• SPERLING, Karin
  D-67433 Neustadt (DE)
• WESTENFELDER, Horst
  D-67435 Neustadt (DE)

(56) Entgegenhaltungen:
DE-A- 2 435 955          FR-A- 2 219 117

• CHEMICAL ABSTRACTS, vol. 116, no. 2,
13.Januar 1992 Columbus, Ohio, US; abstract
no. 8477v, Seite 108; XP000038626 & SU,A,1 646
992 (ZENKOVETS, G.A. ET AL.) 7.Mai 1991

## Beschreibung

Die vorliegende Erfindung betrifft Titandioxid-Pigmente, erhältlich durch vollständige Hydrolyse einer hydrolysierbaren Titanverbindung bei 0 bis 100°C unter intensivem Rühren und unter Einstellung eines pH-Wertes im Bereich von 3 bis 8 und Konstanthaltung dieses pH-Wertes in einem Bereich von 0,3 Einheiten, die mit einer unlöslichen, physiologisch verträglichen, den Photoeffekt von Titandioxid verringernden anorganischen Verbindung beschichtet sind. Außerdem betrifft sie ein Verfahren zur Herstellung solcher beschichteten Titandioxid-Pigmente, ihre Verwendung für kosmetische und medizinische Zwecke, kosmetische Zubereitungen und im wesentlichen wasserfreie Dispersionen, die solche beschichteten Titandioxid-Pigmente enthalten, und ein Verfahren zur Herstellung solcher Dispersionen.

FR-A 2.219.177 betrifft ein Verfahren zur Herstellung von makroporösem hitzestabilen Titanoxid aus Titansulfat und Ammoniumhydroxydlösungen.

DE-A 2435955 beschreibt ein Verfahren zur Herstellung eines Titandioxid-Pigmentes aus einer Titansulfatlösung durch Hydrolyse.

Zeukovets et al. (Chemical Abstracts, vol 116, no. 2, Nr. 8477v, Seite 108, 13.01.1992) beschreiben die Herstellung von Titanoxid durch Hydrolyse von $TiCl_4$.

Die Verwendung von Titandioxid-Pigmenten in Kosmetika, insbesondere Sonnenschutzmitteln, ist allgemein bekannt, beispielsweise aus "Römpp Chemie Lexikon", 9. Aufl., Thieme Verlag, Stuttgart/New York, 1992, S. 4630.

Unter Sonnenschutzmitteln versteht man allgemein Mittel zum Schutz der menschlichen Haut gegenüber schädigenden Einflüssen der Strahlung der Sonne, insbesondere der Ultraviolettstrahlung (UV), die man nach ihrer biologischen Wirkung in die Bereiche UV-A (Wellenlänge 400-320 nm), UV-B (320-280 nm) und UV-C (280-200 nm) unterteilt (Römpp, a.a.O., S. 4804). Ein Sonnenschutzmittel hat speziell die Aufgabe, das für die Bräunung der Haut verantwortliche UV-A unverändert passieren zu lassen, dagegen das UV-B, das Hautschädigungen hervorrufen kann, zurückzuhalten; UV-C wird dagegen in der Atmosphäre weitestgehend absorbiert (Römpp, a.a.O., S. 4213).

Die Herstellung von als Sonnenschutzmittel geeigneten Titandioxid-Pigmenten ist allgemein bekannt, beispielsweise aus der GB-A 2206339.

Dazu schließt man Ilmenit ($FeTiO_3$) mit konzentrierter Schwefelsäure auf, löst den Aufschlußkuchen in Wasser und reduziert das vorhandene Eisen-(III) chemisch zu Eisen-(II). Nach einem Kristallisations- und Filtrationsschritt engt man die Lösung im Vakuum ein und hydrolysiert anschließend in der Hitze zur Ausfällung des Titandioxids. Nach der Neutralisierung der Lösung mit Natronlauge filtriert man das Titandioxid ab und wäscht es mit Wasser nach.

Nach einer Naßmahlung des Titandioxids können die Pigmente gegebenenfalls mit einer anorganischen Verbindung beschichtet werden. Anschließend trocknet man das Produkt bei 110°C.

Die Pigmente können mit Hilfe von Dispergiermitteln und Hochgeschwindigkeits-Kugelmühlen in zahlreichen Lösungsmitteln suspendiert werden.

Derartige Pigmente eignen sich zwar für Sonnenschutzmittel; jedoch weisen sie eine gegenüber der gewünschten UV-B-Absorption unerwünscht hohe UV-A-Absorption und eine niedrige Transparenz im Bereich des sichtbaren Lichts auf.

Der Erfindung lag daher die Aufgabe zugrunde, Titandioxid-Pigmente, die eine gegenüber einer gewünschten UV-B-Absorption geringe UV-A-Absorption und hohe Transparenz im Bereich des sichtbaren Lichts aufweisen, auf technisch einfache und wirtschaftliche Weise herzustellen.

Demgemäß wurden die eingangs definierten Titandioxid-Pigmente gefunden.

Die Titandioxid-Pigmente können durch vollständige Hydrolyse von hydrolysierbaren Titanverbindungen erhalten werden. Als hydrolysierbare Titanverbindungen kommen organische Verbindungen wie Komplexverbindungen, beispielsweise Titanylacetylacetonat und Titanocendichlorid, Titansäureester, beispielsweise Titanmethylat, Titanethylat, Titanpropylat, Titanisopropylat, Titanbutylat und Titankresylat, sowie organische Titansalze, beispielsweise Titanstearylat und Titanoxalat, und vorzugsweise anorganische Verbindungen wie Titanylsulfat, Titanfluorid, Titanbromid, Titanjodid und insbesondere Titantetrachlorid oder Mischungen solcher Verbindungen in Betracht. Derartige Verbindungen sind bekannt.

Bei der Hydrolyse wird erfindungsgemäß ein pH-Wert von 3 bis 8, insbesondere von 3 bis 5, gemessen mit einer Glaselektrode, eingehalten, wobei der pH-Wert während der Hydrolysereaktion erfindungsgemäß höchstens in einem Bereich, bezogen auf den Maximal- und den Minimalwert, von 0,3, vorzugsweise 0,2, Einheiten schwanken sollte.

Der gewünschte pH-Wert der Reaktionsmischung kann in an sich bekannter Weise durch Zugabe von Säuren, Basen oder Puffersystemen eingestellt und innerhalb des erfindungsgemäßen Bereichs gehalten werden.

Als Basen kommen Laugen wie Natronlauge und Ammoniaklösung oder Lösungen basischer Salze wie Natriumcarbonat und Kaliumcarbonat, als Säuren insbesondere Mineralsäuren wie Salzsäure und Schwefelsäure in Betracht.

Die Hydrolyse kann vorteilhaft mit Wasser allein oder mit einem Gemisch aus Wasser und einem mit Wasser mischbaren flüssigen Verdünnungsmittel durchgeführt werden. Als flüssige Verdünnungsmittel eignen sich wasserlösliche Alkohole wie Ethanol und Methanol und wasserlösliche Ether, insbesondere cyclische Ether, wie Tetrahydrofuran und Dioxan.

Die Reaktion wird in der Regel bei einer Temperatur von 0 bis 100°C, bevorzugt 10 bis 30°C, unter intensivem Rühren durchgeführt, wodurch sich Reaktionszeiten von 1 bis 25 Stunden ergeben.

Auf die Titandioxid-Pigmente können anschließend andere anorganische Verbindungen aufgetragen werden. Sollen die beschichteten Titandioxid-Pigmente für medizinische oder kosmetische Zwecke verwendet werden, so kommen insbesondere physiologisch verträgliche, den Photoeffekt von Titandioxid verringernde anorganische Verbindungen wie wasserhaltige Oxide oder Phosphate des Aluminiums, Bors, Siliziums, Zinks, Eisens, Zirkoniums und Cers, Alkali- oder Erdalkaliborate sowie Gemische solcher Verbindungen in Betracht, die nach bekannten Verfahren wie Auffällung in Mengen von 0,1 bis 50 Gew.-%, bezogen auf das Pigment-Gesamtgewicht, aufgetragen werden können.

Zur Entfernung der bei der Herstellung der Titandioxid-Pigmente anfallenden Nebenprodukte empfiehlt sich ein Reinigungsschritt, der in an sich bekannter Weise, beispielsweise durch Filtration und anschließendem Nachwaschen, erfolgen kann.

Die beschichteten und unbeschichteten Titandioxid-Pigmente können aus der wasserhaltigen Suspension nach bekannten Methoden, z.B. durch Filtration und Trocknung, isoliert oder vorteilhaft zur Herstellung der eingangs erwähnten im wesentlichen wasserfreien Dispersionen eingesetzt werden.

Die erfindungsgemäß erhältlichen Titandioxid-Pigmente weisen eine für kosmetische Zwecke besonders vorteilhafte mittlere Partikelgröße (Primärpartikel) von 3 bis 8 nm, insbesondere 3 bis 5 nm und eine spezifische Oberfläche nach DIN 66132, gemessen mit einem Ströhlein-Areameter (Fa. Ströhlein, Düsseldorf) nach dem Einpunkt-Differenzverfahren nach Haul und Dümbgen, von 200 bis 400 m$^2$/g, vorzugsweise 250 bis 350 m$^2$/g auf und können, gemessen mit Elektronenbeugung, amorph oder in einer im wesentlichen aus Anatas bestehenden kristallinen Form vorliegen.

Zur Herstellung der die Titandioxid-Pigmente enthaltenden Dispersionen mischt man die Pigmente oder die wasserhaltige Suspension mit einem nichtwäßrigen Verdünnungsmittel und gegebenenfalls Zusatzstoffen.

Als nichtwäßrige Verdünnungsmittel eignen sich Öle, insbesondere physiologisch unbedenkliche Öle, beispielsweise Fettalkohole, Fettsäuren wie Ölsäure, Fettsäureester wie Isopropylpalmitat, Isopropylstearat, Isopropyllaurat und Isopropylmyristat, Pflanzenöle wie Sonnenblumenöl, Jojobaöl, Erdnußöl, Mandelöl, Avocadoöl, Macadamianußöl, Rizinußöl, Maiskeimöl und Castoröl, Mineralöle wie flüssige Paraffine, Benzoesäureester, insbesondere C$_{12}$-C$_{15}$-Alkylbenzoate, sowie Oligoglycerinester wie Caprylsäuretriglycerid.

Als Zusatzstoffe kommen in erster Linie Dispergierhilfsmittel wie neutrale oder saure Phosphorsäureester oder deren Salze, beispielsweise Diceteareth-10-Phsphorsäureester, Triceteareth--4-Phosphat und Trilaureth-4-Phosphat, primäre, sekundäre, tertiäre und quartäre Ammoniumverbindungen, beispielsweise Cetyldimethyl-2-hydroxyethyl-ammonium-dihydrogenphosphat, Polyacrylate, Carbonsäuren und Hydroxycarbonsäuren sowie deren Salze, Ester, insbesondere mit Sorbit oder Glycerin, beispielsweise Polyoxyethylen-20-Sorbit-Fettsäureester, Metallalkoxide, Alkanolamine und Silikone in Betracht.

Wurde zur Herstellung der Dispersionen die wäßrige Suspension eingesetzt, so kann der Wassergehalt der Dispersion nach bekannten Verfahren wie der Destillation weiter gesenkt werden, so daß praktisch wasserfreie, insbesondere ölige Dispersionen erhalten werden können.

Besondere Bedeutung haben derartige Dispersionen bei der Herstellung von kosmetischen Zubereitungen und Arzneimitteln.

Dazu können den Dispersionen in an sich bekannter Weise weitere Stoffe, insbesondere Stabilisatoren wie Magnesiumsalze und Aluminiumsalze von Fettsäuren, Komplexbildner wie Ethylendiamintetraacetat (EDTA), Antioxidantien wie α-Tocopherol und kosmetische Wirkstoffe wie Panthenol, Bisabolol, α-Tocopherol, α-Tocopherolacetat, Aloe Vera, Algenextrakt und Hyaluronsäure zugesetzt werden.

Die erfindungsgemäßen Titandioxid-Pigmente, Dispersionen, kosmetischen Zubereitungen und Arzneimittel zeichnen sich durch eine im Verhältnis zur UV-B-Absorption geringen UV-A-Absorption und hohen Transparenz im Bereich des sichtbaren Lichts, ausgedrückt durch das Verhältnis des Extinktionskoeffizienten im UV-A- bzw. sichtbaren Bereich zu dem Extinktionskoeffizienten im UV-B-Bereich, aus.

Der Extinktionskoeffizient ist dabei durch folgende Gleichung definiert:

$$A = \varepsilon \cdot C \cdot L$$

mit A:  Extinktion wobei $A = \ln I/I_0$

mit I :  Intensität des Lichts nach Durchgang durch die Probe

I$_0$:  Intensität des eingestrahlten Lichts

ε:  Extinktionskoeffizient [1/g·cm], jeweils bezogen auf die in Klammern angegebene Wellenlänge des Lichts

C:  Konzentration Titandioxid [g/l]

L:     Weglänge [cm].

Für die drei Bereiche wurde mit einem UV-VIS-Spektrometer (Gerät HP 8452, Fa. Hewlett-Packard) bei folgenden Wellenlängen gemessen:

Sichtbares Licht:          400 nm
UV-A-Bereich:              380 nm
UV-B-Bereich:              308 nm.

Als Maß für eine gegenüber dem UV-A- und sichtbaren Bereich hohe UV-B-Absorption dient dabei das Verhältnis $\varepsilon(308)/\varepsilon(380)$ und $\varepsilon(308)/\varepsilon(400)$.

Beispiel 1

380 g Titantetrachlorid und 1000 ml Natronlauge (NaOH-Gehalt 320 g/l) wurden synchron zu 3800 g Wasser unter intensivem Rühren bei 20°C innerhalb von 60 min bei einem pH-Wert von 3 zugegeben und weitere 30 min nachgerührt. Anschließend wurde unter intensivem Rühren mit 25 Gew.-%-iger Natronlauge ein pH-Wert von 8 eingestellt und nochmals 30 min nachgerührt.
Nach dem Abfiltrieren wurde der Filterkuchen mit 9 1 destilliertem Wasser nachgewaschen.
Die bei 100°C/20 mbar getrockneten Titandioxid-Pigmente wiesen eine mittlere durchschnittliche Partikelgröße von 5 nm und eine spezifische Oberfläche von 253 m$^2$/g auf.
Zu 461 g des Filterkuchens, der einen Titandioxid-Gehalt von 21,1 Gew.-% aufwies, wurde eine Lösung von 83,7 g Cetyl-dimethyl-2-hydroxyethyl-ammonium-dihydrogenphosphat in 195,3 g Wasser gegeben. Nach 30 min intensivem Rühren wurden 400 g Caprylsäuretriglycerid zugegeben, wonach das Wasser bei 80°C/40-50 mbar abdestilliert wurde.
An einer mit weiterem Caprylsäuretriglycerid auf einen Feststoff-Gehalt von 50 mg/l verdünnten Probe wurden die in Tabelle 1 aufgeführten Extinktionskoeffizienten gemessen.

Beispiel 2

760 g Titantetrachlorid und 2000 ml Natronlauge (NaOH-Gehalt 320 g/l) wurden synchron zu 3800 g Wasser unter intensivem Rühren bei 20°C innerhalb von 120 min bei einem pH-Wert von 3 zugegeben, und weitere 90 min bei 100°C gerührt. Anschließend wurde bei 20°C unter intensivem Rühren mit 25 Gew.-%iger Natronlauge ein pH-Wert von 8 eingestellt und 30 min bei 20°C nachgerührt.
Nach dem Abfiltrieren wurde der Filterkuchen mit 9 l destilliertem Wasser nachgewaschen.
Die bei 100°C/20 mbar getrockneten Titandioxid-Pigmente wiesen eine mittlere durchschnittliche Partikelgröße von 5 nm und eine spezifische Oberfläche von 286 m$^2$/g auf.
Zu 333,2 g des Filterkuchens, der einen Titandioxid-Gehalt von 26,5 Gew.-% aufwies, wurde wurde eine Lösung von 35,3 g Cetyl-dimethyl-2-hydroxyethyl-ammonium-dihydrogenphosphat in 82,3 g Wasser gegeben. Nach 30 min intensivem Rühren wurden 233 g Caprylsäuretriglycerid zugegeben, wonach das Wasser bei 80°C/40-50 mbar abdestilliert wurde.
An einer gemäß Beispiel 1 verdünnten Probe wurden die in Tabelle 1 aufgeführten Extinktionskoeffizienten gemessen.

Beispiel 3

380 g Titantetrachlorid und 1000 ml Natronlauge (NaOH-Gehalt 320 g/l) wurden synchron zu 3800 g Wasser unter intensivem Rühren bei 20°C innerhalb von 90 min bei einem pH-Wert von 5 zugegeben. Anschließend wurde 30 min nachgerührt, innerhalb von 15 min 180 ml einer wäßrigen Lösung von Al$_2$(SO$_4$)$_3$*18 H$_2$O (Gehalt: 666,4 g/l) unter intensivem Rühren zugegeben. Dabei wurde mit 40 ml Natronlauge (NaOH-Gehalt 320 g/l) konstant ein pH-Wert von 5 gehalten. Anschließend wurde mit 25 Gew.-%iger Natronlauge ein pH-Wert von 8 eingestellt und nochmals 30 min nachgerührt.
Nach dem Abfiltrieren wurde der Filterkuchen mit 11 1 destilliertem Wasser nachgewaschen.
Die bei 100°C/20 mbar getrockneten Titandioxid-Pigmente wiesen eine mittlere durchschnittliche Partikelgröße von 5 nm und eine spezifische Oberfläche von 296 m$^2$/g auf.
Zu 1000 g des Filterkuchens, der einen Feststoffgehalt von 16,4 Gew.-% aufwies, wobei der Feststoff zu 78,4 Gew.-% aus Titandioxid bestand, wurden 97,1 g Hostaphat KL 340 N (Firma Hoechst AG) und 246 g Caprylsäuretriglycerid gegeben und 8 Stunden intensiv gerührt, wonach das Wasser bei 70°C / 40-50 mbar abdestilliert wurde.

An einer gemäß Beispiel 1 verdünnten Probe wurden die in Tabelle 1 aufgeführten Extinktionskoeffizienten gemessen.

Tabelle 1

| Probe | $\varepsilon(308)$ | $\varepsilon(380)$ | $\varepsilon(400)$ | $\varepsilon(308)/\varepsilon(380)$ | $\varepsilon(308)/\varepsilon(400)$ |
|---|---|---|---|---|---|
| Bsp. 1 | 30,1 | 13,9 | 12,1 | 2,17 | 2,49 |
| Bsp. 2 | 34,0 | 18,2 | 15,8 | 1,87 | 2,15 |
| Bsp. 3 | 36,3 | 15,3 | 14,0 | 2,37 | 2,59 |

**Patentansprüche**

1. Mit einer unlöslichen, physiologisch verträglichen, den Photoeffekt von Titandioxid verringernden anorganischen Verbindung in einer Menge von 0,1 bis 50 Gew.-%, bezogen auf das Pigment-Gesamtgewicht, beschichtete Titandioxid-Pigmente, erhältlich durch vollständige Hydrolyse einer hydrolisierbaren Titanverbindung bei 0 bis 100°C unter intensivem Rühren und unter Einstellung eines pH-Werts im Bereich von 3 bis 8 und Konstanthaltung dieses pH-Wertes in einem Bereich von 0,3 Einheiten und anschließendes Auffällen der anorganischen Verbindung in an sich bekannter Weise.

2. Verfahren zur Herstellung der Titandioxid-Pigmente gemäß Anspruch 1, dadurch gekennzeichnet, daß man eine hydrolysierbare Titanverbindung unter intensivem Rühren und unter Einstellung eines pH-Wertes im Bereich von 3 bis 8 und anschließender Konstanthaltung des pH-Wertes in einem Bereich von 0,3 Einheiten bei 0 bis 100°C vollständig hydrolysiert und anschließend eine physiologisch verträgliche, den Photoeffekt von Titandioxid verringernde anorganische Verbindung in sich bekannter Weise auffällt.

3. Verwendung der Titandioxid-Pigmente gemäß Anspruch 1 für kosmetische Zwecke.

4. Kosmetische Zubereitungen, enthaltend ein Titandioxid-Pigment gemäß Anspruch 1 für die äußere Anwendung am menschlichen und tierischen Körper.

5. Im wesentlichen wasserfreie Dispersionen, enthaltend ein Titandioxid-Pigment gemäß Anspruch 1.

6. Verfahren zur Herstellung von Dispersionen gemäß Anspruch 5, dadurch gekennzeichnet, daß man eine hydrolysierbare Titanverbindung unter intensivem Rühren und unter Einstellung eines pH-Wertes im Bereich von 3 bis 8 und Konstanthaltung des pH-Wertes in einem Bereich von 0,3 Einheiten bei 0 bis 100°C vollständig hydrolysiert, die Titandioxid-Pigmente gegebenenfalls mit einer anorganischen Verbindung in an sich bekannter Weise beschichtet, abtrennt und anschließend die Titandioxid-Pigmente in einem im wesentlichen nichtwäßrigen flüssigen Verdünnungsmittel dispergiert.

7. Verwendung der Dispersionen gemäß Anspruch 5 zur Herstellung kosmetischer Zubereitungen.

8. Titandioxid-Pigmente gemäß Anspruch 1 zur Verwendung als Arzneimittel.

9. Verwendung von Titandioxid-Pigmenten gemäß Anspruch 1 zur Herstellung von Arzneimitteln zur Behandlung von Verbrennungen der Haut oder von Sonnenallergien.

**Claims**

1. A titanium dioxide pigment coated with an insoluble, physiologically tolerable inorganic compound reducing the photoeffect of titanium dioxide and in an amount from 0.1 to 50% by weight, based on the total pigment weight, obtainable by complete hydrolysis of a hydrolyzable titanium compound at from 0 to 100°C with intensive stirring and with setting of a pH in the range from 3 to 8 and maintenance of this pH within a range of 0.3 units and subsequent precipitation of the inorganic compound in a manner known per se.

2. A process for the preparation of the titanium dioxide pigments as claimed in claim 1, which comprises completely hydrolyzing a hydrolyzable titanium compound with intensive stirring and with setting of a pH in the range from 3 to 8 and subsequent maintenance of the pH within a range of 0.3 units at from 0 to 100°C and then precipitating a physiologically tolerable inorganic compound reducing the photoeffect of titanium dioxide in a manner known per se.

3. The use of the titanium dioxide pigments as claimed in claim 1 for cosmetic purposes.

4. A cosmetic preparation comprising a titanium dioxide pigment as claimed in claim 1 for external application to the human or animal body.

5. An essentially anhydrous dispersion comprising a titanium dioxide pigment as claimed in claim 1.

6. A process for the preparation of dispersions as claimed in claim 5, which comprises completely hydrolyzing a hydrolyzable titanium compound with intensive stirring and with setting of a pH in the range from 3 to 8 and maintenance of the pH within a range of 0.3 units at from 0 to 100°C, coating the titanium dioxide pigment, if desired, with an inorganic compound in a manner known per se, separating it off and then dispersing the titanium dioxide pigment in an essentially nonaqueous liquid diluent.

7. The use of the dispersions as claimed in claim 5 for the production of cosmetic preparations.

8. A titanium dioxide pigment as claimed in claim 1 for use in medicaments.

9. The use of titanium dioxide pigments as claimed in claim 1 for the production of medicaments for the treatment of burns to the skin or of sun allergies.


**Revendications**

1. Dioxyde de titane pigmentaire revêtu d'un composé inorganique insoluble, acceptable pour les usages pharmaceutiques, amoindrissant l'effet photo du dioxyde de titane, en quantité de 0,1 à 50 % du poids total du pigment, qu'on obtient par hydrolyse totale d'un dérivé hydrolysable du titane à des températures de 0 à 100°C sous agitation intensive et avec réglage à un pH dans l'intervalle de 3 à 8, ce pH étant maintenu constant dans un intervalle de 0,3 unité, en faisant suivre de la précipitation du composé inorganique sur le pigment, de manière connue en soi.

2. Procédé pour la préparation de dioxyde de titane pigmentaires selon la revendication 1, caractérisé par le fait que l'on hydrolyse totalement un dérivé hydrolysable du titane sous agitation intensive et avec réglage à un pH dans l'intervalle de 3 à 8 maintenu ensuite constant dans un intervalle de 0,3 unité, à des températures de 0 à 100°C, puis on précipite sur le pigment, de manière connue en soi, un composé inorganique acceptable pour les usages pharmaceutiques et amoindrissant l'effet photo du dioxyde de titane.

3. Utilisation des dioxydes de titane pigmentaires selon la revendication 1 dans des applications cosmétiques.

4. Compositions cosmétiques contenant un dioxyde de titane pigmentaire selon la revendication 1, pour l'usage externe sur le corps humain ou animal.

5. Dispersions essentiellement anhydres, contenant un dioxyde de titane pigmentaire selon la revendication 1.

6. Procédé pour la préparation de dispersions selon la revendication 5, caractérisé par le fait que l'on hydrolyse totalement un dérivé hydrolysable du titane sous agitation intensive et avec réglage à un pH dans l'intervalle de 3 à 8, maintenu constant dans un intervalle de 0,3 unité, à des températures de 0 à 100°C, on revêt le cas échéant les dioxydes de titane pigmentaires par un composé inorganique de manière connue en soi, on sépare puis on disperse les dioxydes de titane pigmentaires dans un diluant liquide essentiellement non aqueux.

7. Utilisation des dispersions selon la revendication 5 pour la préparation de compositions cosmétiques.

8. Dioxydes de titane pigmentaires selon la revendication 1, pour l'utilisation en tant que médicaments.

9. Utilisation des dioxydes de titane pigmentaires selon la revendication 1 pour la préparation de médicaments prévus pour le traitement des brûlures de la peau ou des allergies au soleil.